# EUROPÄISCHE PATENTSCHRIFT

(11) **EP 1 427 702 B1**
(45) Veröffentlichungstag und Bekanntmachung des Hinweises auf die Patenterteilung: **14.06.2006**
(21) Anmeldenummer: 02797938.4
(22) Anmeldetag: 30.08.2002
(51) Int. Cl.: C07D 207/26, C07D 201/08

(54) **VERFAHREN ZUR HERSTELLUNG VON 2-PYRROLIDON**
METHOD FOR THE PRODUCTION OF 2-PYRROLIDONE
PROCEDE DE PRODUCTION DE 2-PYRROLIDONE

(30) Priorität: 07.09.2001 DE 10143824
(43) Veröffentlichungstag der Anmeldung: 16.06.2004
(73) Patentinhaber: BASF Aktiengesellschaft, 67056 Ludwigshafen (DE)
(72) Erfinder: RUDLOFF, Martin, 67273 Weisenheim (DE); STOPS, Peter, 67122 Altrip (DE); HENKES, Erhard, 64683 Einhausen (DE); SCHMIDTKE, Helmut, 64625 Bensheim (DE); FISCHER, Rolf-Hartmuth, 69121 Heidelberg (DE); JULIUS, Manfred, 67117 Limburgerhof (DE); LEBKÜCHER, Rolf, 68165 Mannheim (DE); ROSS, Karl-Heinz, 67269 Grünstadt (DE)
(86) Internationale Anmeldenummer: PCT/EP2002/009721
(87) Internationale Veröffentlichungsnummer: WO 2003/022811

(56) Entgegenhaltungen:
- DE-A- 1 795 007
- US-A- 3 133 085
- US-A- 5 393 888
- DATABASE CAPLUS [Online] CHEMICAL ABSTRACTS SERVICE, COLUMBUS, OHIO, US; SIRETEANU, DORINA MARIA RUXANDA ET AL: "Stable high purity pyrrolidinone" retrieved from STN Database accession no. 93:186154 XP002225600 in der Anmeldung erwähnt & RO 65 067 B (COMBINATUL PETROCHIMIC "BORZESTI", ROM.) 15. Februar 1979 (1979-02-15)
- DATABASE CAPLUS [Online] CHEMICAL ABSTRACTS SERVICE, COLUMBUS, OHIO, US; SHIMODAIRA, TAKASHI ET AL: "2-Pyrrolidinone" retrieved from STN Database accession no. 83:9774 XP002225601 & JP 49 117460 A (MITSUBISHI PETROCHEMICAL CO., LTD.) 9. November 1974 (1974-11-09)

## Beschreibung

Die vorliegende Erfindung betrifft ein Verfahren zur kontinuierlichen Herstellung von 2-Pyrrolidon durch Umsetzung von gamma-Butyrolacton mit Ammoniak in der Flüssigphase in Gegenwart von Wasser.

2-Pyrrolidon (gamma-Butyrolactam) ist ein wichtiges Lösungsmittel, Extraktionsmittel und Zwischenprodukt zur Herstellung von N-Vinylpyrrolidon und Pharmaceutika (Ullmann's Encyclopedia of Industrial Chemistry, 5^{th} Ed., Vol. A22, Seiten 457 bis 463).

In Chemische Berichte 69, Seite 2727-31, 1936, wird die diskontinuierliche Umsetzung von GBL mit wasserfreiem Ammoniak bei 200°C unter Eigendruck beschrieben. Die Umsetzung lieferte 2-Pyrrolidon in einer Ausbeute von 64 %.

US-A-4,824,967 (GAF Corp.) beschreibt die Synthese von 2-Pyrrolidon durch Umsetzung von gamma-Butyrolacton (GBL) mit Ammoniak in der Gasphase an einem Magnesiumsilikat-Katalysator bei 230 bis 300°C, bevorzugt 250 bis 290°C, und einem Druck von 50 bis 300 psig (3,5 bis 20,7 bar).

Chem. Abstracts 93(19): 186154b (RO-A-65067, Combanitul Petrochimic) beschreibt die diskontinuierliche Umsetzung von GBL mit Ammoniak im Molverhältnis von 1:1,1 bis 1:1,2 bei 270 bis 330°C und 70 bis 150 atm (70,9 bis 152,0 bar).

DE-A-17 95 007 (BASF AG) beschreibt ein kontinuierliches Verfahren zur Herstellung von 2-Pyrrolidon aus GBL und Aminoniak in der Flüssigphase, wobei die Umsetzung bei Temperaturen zwischen 180 und 340°C und einem Druck, der mindestens 10 %, vorzugsweise mindestens 20 %, oberhalb des Eigendrucks des Reaktionsgemisches bei Reaktionstemperatur liegt.
Die Reaktionsdrücke liegen im allgemeinen zwischen 25 und 280 at., vorzugsweise zwischen 45 und 130 at. (1 at 0,98066 bar).

Die Reaktionstemperaturen betragen vorzugsweise 190 bis 290°C. Die erfindungsgemäßen Beispiele der DE-A-17 95 007 wurden bei 270°C, einem Druck von 70 bis 200 at. und GBL : Ammoniak : Wasser - Molverhältnissen von 1 : 5,6 : 2,4 (Beispiel 1), 1 : 5,3 : 2,4 (Beispiel 2) und 1 : 5,4 : 4,8 (Beispiel 3) durchgeführt.

Derwent Abstract Nr. 95-176475/23 (RO-B1-108 561, SC Chimcomplex SA), betrifft die kontinuierliche Herstellung von 2-Pyrrolidon durch Umsetzung von GBL mit wässrigem Ammoniak in flüssiger Phase in zwei Stufen, wobei die erste Stufe bei 40 bis 45°C und 1 atm und die zweite Stufe bei 270 bis 280°C und 80 bis 90 bar durchgeführt wird.

US-A 5,393,888 (ISP Investments Inc.) beschreibt die Umsetzung von GBL mit Ammoniak zu 2-Pyrrolidon bei Molverhältnissen von GBL : Ammoniak von 1: 0,5 bis 0,85, Temperaturen von 200 bis 375°C und Drucken von 700 bis 1800 psi (48,3 bis 124,1 bar).

WO 99/52866 (Pantochim) offenbart ein Verfahren zur kontinuierlichen Herstellung von 2-Pyrrolidon durch Umsetzung von GBL mit Ammoniak in der Flüssigphase in drei aufeinanderfolgenden Reaktionsstufen, wobei die erste Stufe bei Temperaturen zwischen 130 und 200°C, die zweite Stufe bei Temperaturen zwischen 200 und 250°C und die dritte Stufe bei Temperaturen zwischen 250 und 320°C, betrieben wird. Der Druck in allen drei Stufen liegt zwischen 40 und 120 ATE (40,53 und 121,59 bar), bevorzugt zwischen 60 und 100 ATE (60,80 und 101,33 bar).

US-A-4,014,900 (S.F. Pusztaszeri) betrifft ein Verfahren zur Reinigung von kommerziellem 2-Pyrrolidon durch Behandlung mit einem Metallhydroxid und Flashverdampfung von einer erhitzten Oberfläche bei bestimmten Temperaturen und Drucken.

Erfindungsgemäß wurde erkannt, dass sich aus dem Stand der Technik unter anderem folgende Nachteile ergeben:
a) die geringe Raum-Zeit-Ausbeute und Notwendigkeit eines Katalysators bei Durchführung der Umsetzung in der Gasphase,
b) der große molare Überschuss an Ammoniak bezogen auf GBL, der einen hohen technischen Aufwand bei der Aufarbeitung des Reaktionsaustrags und Rückgewinnung des unumgesetzten Ammoniaks und damit hohe Investitions- und Energiekosten bedingt, bei der Fahrweise gemäß DE-A-17 95 007 und
c) der hohe technische Aufwand verbunden mit hohen Investitions- und Betriebskosten bei einer Durchführung der Umsetzung in mehreren Reaktionsstufen, z.B. gemäß WO 99/52866.

Der vorliegenden Erfindung lag die Aufgabe zugrunde, unter Überwindung der Nachteile des Stands der Technik, ein verbessertes, selektives Verfahren zur Herstellung von 2-Pyrrolidon in hohen Ausbeuten und Raum-Zeit-Ausbeuten und hoher Qualität (z.B. Reinheit > 99,5 GC-Fl.%, APHA-Farbzahl ≤ 30) aufzufinden.

Demgemäß wurde ein Verfahren zur kontinuierlichen Herstellung von 2-Pyrrolidon durch Umsetzung von gamma-Butyrolacton mit Ammoniak in der Flüssigphase in Gegenwart von Wasser gefunden, welches dadurch gekennzeichnet ist, dass man die Umsetzung bei einer Temperatur von 275 bis 300°C und einem Absolutdruck von 140 bis 180 bar durchführt.

Vorzugsweise wird die erfindungsgemäße Umsetzung bei einer Temperatur von 280 bis 295°C, insbesondere bei einer Temperatur von 280 bis 290°C, ganz besonders bei einer Temperatur von 282 bis 288°C, z.B. 285°C, durchgeführt.

Die erfindungsgemäße Umsetzung wird vorzugsweise bei einem Absolutdruck von 150 bis 170 bar, insbesondere bei einem Absolutdruck von 155 bis 165 bar, z.B. 160 bar, durchgeführt.

Das Molverhältnis der Einsatzstoffe GBL : Ammoniak : Wasser beträgt im erfindungsgemäßen Verfahren im allgemeinen 1 : (1,5 bis 5) : (0,5 bis 4), bevorzugt 1 : (2 bis 4) : (1 bis 3), insbesondere 1 : (2,1 bis 3,5) : (1,5 bis 2,8), ganz besonders 1 : (2,2 bis 3) : (1,6 bis 2,3), z.B. 1 : 2,6 : 1,9.

Das erfindungsgemäße Verfahren kann z.B. wie folgt ausgeführt werden:

Als Reaktor dient vorzugsweise ein senkrecht stehendes, schlankes Hochdruckrohr, das am oberen Ende mit einem über einen Druckregler gesteuertes Entspannungsventil ausgestattet ist. Die Feedströme werden vorzugsweise vom Reaktoraustrag vorgeheizt, so dass im Reaktor durch die Exothermie die erfindungsgemäßen Reaktionstemperaturen eingestellt werden können. Im Reaktor sind bevorzugt mehrere Siebböden zur Verhinderung einer Rückvermischung und besseren Ausbildung eines Pfropfenströmung (quasi Rührkesselkaskade) eingebaut. Die Anzahl der Siebböden kann 10 bis 30, bevorzugt 12 bis 24, betragen.

Flüssiges Ammoniak, gegebenenfalls gemischt mit zurückgewonnenen Ammoniak aus der Aufarbeitung (siehe unten), wird mit einer Pumpe, z.B. Membranpumpe, zu einem dampfbetriebenen Aufheizer W1 gefördert.

Wasser, gegebenenfalls gemischt mit zurückgewonnenem Wasser aus der Aufarbeitung (siehe unten), wird mit einer Pumpe, z.B. Kolbenpumpe, ebenfalls zum Aufheizer W1 gefördert.

Das flüssige Ammoniak/Wasser-Gemisch wird vom W1 über einen Wärmetauscher W3 an das untere Ende des Rohrreaktors geführt (bevorzugte Sumpffahrweise).

GBL wird mit einer Pumpe, z.B. Membranpumpe, über einen dampfbetriebenen Aufheizer W2 ebenfalls an das untere Ende des Rohrreaktors geführt, wobei eine Durchmischung der Einsatzstoffe stattfindet und die Einsatzstoffe GBL, Ammoniak und Wasser im oben angegebenen, erfindungsgemäßen Molverhältnis vorliegen.

Das GBL sowie das Ammoniak/Wasser-Gemisch werden in einer besonders bevorzugten Ausführungsform dem Rohrreaktor im Zentrum des Reaktorbodens durch einen Zweistoff-Injektor separat zugeführt. Während das GBL hierbei mit hoher Strömungsgeschwindigkeit (bevorzugt 4 - 12 m/s) durch die Zentraldüse eingeleitet wird, gelangt das Ammoniak/Wasser-Gemisch an der Außenseite dieser Düse durch einen Ringspalt in den Reaktor. Die Edukte werden hierbei als Treibstrahl in ein Umlaufrohr geleitet, das sich im Eintrittsbereich des Reaktors befindet, ca. ein Drittel des freien Reaktorquerschnittes umschließt und eine Länge von bevorzugt 1,5 bis 2,0 m besitzt. Die Rezirkulation von Flüssigkeit, die an der Außenseite des Umlaufrohres erfolgt und durch den Treibstrahl in Gang gehalten wird, führt zu einem intensiven ersten Kontakt der Reaktionspartner im oben angegebenen, erfindungsgemäßen Molverhältnis.

Im Reaktor erfolgt unter den oben angegebenen Temperaturen und Drucken die kontinuierliche Umsetzung des GBL mit Ammoniak in flüssiger Phase, in Gegenwart von Wasser und in exothermer Reaktion zu 2-Pyrrolidon.

Die Umsetzung wird bevorzugt in Abwesenheit eines Katalysators durchgeführt.

Die mittleren Verweilzeiten des Reaktionsgemischs im Reaktor, ermittelt mit der Dichte von GBL, Ammoniak (flüssig) und Wasser bei Normaltemperatur (20°C), betragen je nach Belastung im allgemeinen 20 bis 60 Min., bevorzugt 30 bis 45 Min.

Das erhaltene Reaktionsprodukt wird aus dem Reaktor kontinuierlich zu einer Destillationskolonne K1 entspannt, wobei der Reaktoraustrag zunächst zur Aufheizung des Ammoniak/Wasser-Gemischs ganz oder teilweise über den Wärmetauscher W3 (siehe oben) geleitet und dabei abgekühlt wird.

Das überschüssige Ammoniak wird in der Destillationskolonne K1, z.B. bei 40 bis 160°C und 15 bis 18 bar, abdestilliert, wobei die Wärmezufuhr mit einem Umlaufverdampfer erfolgt, und kann in die Synthese zurückgeführt werden.

Bevorzugt erfolgt die Abdestillation des Ammoniaks in dieser Destillationsstufe in Gegenwart von 0,1 bis 1 Gew.-%, insbesondere 0,5 bis 0,9 Gew.-%, (jeweils bezogen auf die Menge an 2-Pyrrolidon im Zulauf dieser Destillationsstufe) Hydroxiden der Metalle Na, K, Li, Ba oder Ca.

Besonders bevorzugt erfolgt die Abdestillation des Ammoniaks in dieser Destillationsstufe in Gegenwart von NaOH, das als Natronlauge mittels einer Pumpe in den Sumpf dieser Kolonne dosiert wird. z.B. wird hier wässrige 25 %ige Natronlauge so eingesetzt, dass die Konzentration an NaOH 0,1 bis 1 Gew.-%, insbesondere 0,5 bis 0,9 Gew.-%, (jeweils bezogen auf die Menge an 2-Pyrrolidon im Zulauf dieser Destillationsstufe) beträgt.

Erfindungsgemäß wurde erkannt, dass durch diese besondere Verfahrensausgestaltung im Reaktionsprodukt enthaltenes CO₂ gebunden wird, die Carbamat- und Carbaminat-Bildung im Kopf der Kolonne K1 unterbunden wird und restliche Spuren an unumgesetzten GBL zum entsprechenden Metallsalz der gamma-Hydroxybuttersäure umgesetzt werden, so dass schließlich ein besonders reines 2-Pyrrolidon erhalten wird.

Anschließend wird der ammoniakfreie Sumpf in eine weitere Destillationskolonne entspannt, wo, z.B. bei 0,1 bis 0,3 bar, insbesondere bei 0,15 bis 0,2 bar, das Wasser abdestilliert wird, das teilweise in die Synthese zurückgeführt werden kann. Die Wärmezufuhr kann auch hier mit einem Umlaufverdampfer erfolgen.

Das so im Sumpf dieser Kolonne erhaltene rohe 2-Pyrrolidon, das als Nebenprodukte geringe Mengen an gamma-(N-2-Pyrrolidonyl)-buttersäureamid und gamma-(N-2-Pyrrolidonyl)-buttersäure enthalten kann, wird dann in einer weiteren Destillationskolonne, z.B. bei Drucken von 1 bis 2 mbar, reindestilliert. (Siedepunkt von 2-Pyrrolidon: 250°C/1013 mbar).

Nach dem erfindungsgemäßen Verfahren wird 2-Pyrrolidon in Ausbeuten > 93 %, insbesondere > 94 % erhalten. Der Umsatz an GBL beträgt im allgemeinen > 98 %, insbesondere > 99 %, ganz besonders > 99,9 %. Die Selektivität (bezogen auf GBL) beträgt im allgemeinen > 93 %, insbesondere > 94%.

Das erfindungsgemäß erhaltene 2-Pyrrolidon besitzt eine hohe Qualität:

Die Reinheit beträgt im allgemeinen > 99,5 GC-F1.%, insbesondere ≥ 99,8 GC-Fl.% (GC-Bedingungen: 25 m CP-WAX 52 CB, Temperaturprogramm: 140°C/8 Min. Haltezeit, 5°C/Min. Aufheizrate, 180°C Endtemperatur/15 Min. Haltezeit). Der Gehalt an 1,4-Butandiol beträgt im allgemeinen < 0,1 GC-F1.%, insbesondere < 0,07 GC-F1.%, der Gehalt an GBL < 0,1 GC-F1.%, insbesondere < 0,05 GC-F1.% (GC-Bedingungen wie oben). Der Gehalt an 3- und 4-Methyl-2-pyrrolidon beträgt im allgemeinen jeweils < 0,1 GC-F1.%, insbesondere jeweils < 0,08 GC-F1.% (GC-Bedingungen wie oben).

Die APHA-Farbzahl nach DIN ISO 6271 des erfindungsgemäß erhaltenen 2-Pyrrolidons beträgt im allgemeinen ≤ 30, insbesondere < 5.

Das erfindungsgemäße Verfahren kann auch in einer Apparatur, wie sie in der Schrift DE-A-17 95 007, auf die hier diesbezüglich ausdrücklich Bezug genommen wird, beschrieben ist, ausgeführt werden.

Das erfindungsgemäße Verfahren wird bevorzugt einstufig, d.h. in einem Reaktor durchgeführt.

In einer besonderen Ausgestaltung des Verfahrens können auch mehrere Rohrreaktoren (z. B. zwei oder drei Reaktoren, jeweils wie oben beschrieben) in Sumpffahrweise hintereinander geschaltet werden, wobei mindestens in einem dieser Reaktoren, bevorzugt im letzten dieser Reaktoren, die erfindungsgemäßen Reaktionsbedingungen herrschen.

Ein Beispiel für eine solche Reihenschaltung von mehreren Reaktoren findet sich in der Schrift WO 99/52866 (siehe dort Fig. 1, Reaktoren Nr. 5, 9 und 13 und die Beschreibung), auf die hier diesbezüglich ausdrücklich Bezug genommen wird.

### Beispiele

### Beispiel 1

Die 2-Pyrrolidon-Synthese wurde unter den unten genannten Bedingungen in kontinuierlicher Fahrweise in einem 1 1-Reaktor (RA4, Länge x Innendurchmesser = 2000 mm x 30 mm, Ringspalt 9 mm, Volumen = 1,1 1, elektrisch beheizt) durchgeführt. Der Reaktor wurde dabei in Sumpffahrweise im geraden Durchgang betrieben. Die Einsatzstoffe Ammoniak und Wasser (als Gemisch) sowie GBL wurden vorgeheizt und mittels Pumpen zum Reaktoreingang gefördert, wo die Mischung der beiden Ströme erfolgte.

Am Reaktorausgang befand sich ein Hochdruckabscheider aus welchem die Gasphase (Stickstoff und Inerte) als Abgas entspannt wurde. Die sich abscheidende Flüssigphase wurde in ein Gefäß auf Atmosphärendruck entspannt, wobei der im Überschuss eingesetzte und nach der Reaktion verbliebene Ammoniak abgaste.

| | |
|---|---|
| Temperatur: | 285 °C |
| Druck: | 160 bar (Druck durch N₂ gehalten) |
| Belastung: | 0,74 kg GBL/ (l_{Reaktor}*h) |
| GBL: NH₃ : H₂O - Molverhältnis | 1: 2,6: 1,9 |
| Abgas | 20 Nl/h |

[Nl = Normliter = auf Normalbedingungen umgerechnetes Volumen; Reaktorbelastung in kg GBL pro Liter Reaktorvolumen und Stunde].

In der folgenden Tabelle ist die Zusammensetzung der Reaktionsausträge (nach Ammoniak-Abdampfung) aufgeführt.

**Tabelle GC-Analyse der Rohausträge aus der 2-Pyrrolidon-Synthese**

| Versuch | 2-Pyrrolidon | GBL | Amid *) | Sonstige |
|---|---|---|---|---|
| Nr. | [GC-Fl.%] | [GC-Fl.%] | [GC-Fl.%] | [GC-Fl.%] |
| 1 | 93,9 | 0,05 | 4,3 | 1,7 |
| 2 | 94,3 | 0,02 | 4,2 | 0,4 |

| | | | | |
|---|---|---|---|---|
| GC-Bedingungen: 30 m RTX 5 Amin, Temperaturprogramm: 80°C Eingangstemperatur, 8°C/Min. Aufheizrate, 280°C Endtemperatur/15 Min. Haltezeit. Der H₂O-Gehalt ist nicht berücksichtigt. | | | | |
| (*) Amid = gamma-(N-2-Pyrrolidonyl)-buttersäureamid) | | | | |

## Patentansprüche

1. Verfahren zur kontinuierlichen Herstellung von 2-Pyrrolidon durch Umsetzung von gamma-Butyrolacton mit Ammoniak in der Flüssigphase in Gegenwart von Wasser, **dadurch gekennzeichnet, dass** man die Umsetzung bei einer Temperatur von 275 bis 300°C und einem Absolutdruck von 140 bis 180 bar durchführt und das Molverhältnis der Einsatzstoffe gamma-Butyrolacton, Ammoniak und Wasser 1 : (2 bis 4) : (1 bis 3) beträgt.

2. Verfahren nach Anspruch 1, **dadurch gekennzeichnet, dass** man die Umsetzung bei einer Temperatur von 280 bis 295°C durchführt.

3. Verfahren nach einem der vorhergehenden Ansprüche, **dadurch gekennzeichnet, dass** man die Umsetzung bei einem Absolutdruck von 150 bis 170 bar durchführt.

4. Verfahren nach einem der vorhergehenden Ansprüche, **dadurch gekennzeichnet, dass** das Molverhältnis der Einsatzstoffe gamma-Butyrolacton, Ammoniak und Wasser 1: (2,1 bis 3,5) : (1,5 bis 2,8) beträgt.

5. Verfahren nach einem der vorhergehenden Ansprüche, **dadurch gekennzeichnet, dass** man die Umsetzung einstufig durchführt.

6. Verfahren nach einem der vorhergehenden Ansprüche, **dadurch gekennzeichnet, dass** man die Umsetzung in einem aufrecht stehenden Rohrreaktor durchführt.

7. Verfahren nach dem vorhergehenden. Anspruch, **dadurch gekennzeichnet, dass** man die Umsetzung in Sumpffahrweise durchführt.

8. Verfahren nach einem der beiden vorhergehenden Ansprüche, **dadurch gekennzeichnet, dass** man das gamma-Butyrolacton und ein Ammoniak/Wasser-Gemisch dem Rohrreaktor im Reaktorboden durch einen Zweistoff-Injektor separat zuführt.

9. Verfahren nach einem der vorhergehenden Ansprüche, **dadurch gekennzeichnet, dass** man die Umsetzung in Abwesenheit eines Katalysators durchführt.

10. Verfahren nach einem der vorhergehenden Ansprüche, **dadurch gekennzeichnet, dass** man nach der Umsetzung aus dem Reaktionsprodukt zunächst überschüssiges Ammoniak, dann Wasser und schließlich das 2-Pyrrolidon abdestilliert.

11. Verfahren nach einem der vorhergehenden Ansprüche, **dadurch gekennzeichnet, dass** man nach der Umsetzung aus dem Reaktionsprodukt das überschüssige Ammoniak in Gegenwart von Hydroxiden der Metalle Na, K, Li, Ba oder Ca, insbesondere in Gegenwart von NaOH, abdestilliert.

12. Verfahren nach einem der vorhergehenden Ansprüche zur Herstellung von 2-Pyrrolidon mit einer Selektivität von > 93 %.

13. Verfahren nach einem der vorhergehenden Ansprüche zur Herstellung von 2-Pyrrolidon mit einer Reinheit von > 99,5 %.

14. Verfahren nach einem der vorhergehenden Ansprüche zur Herstellung von 2-Pyrrolidon mit einer APHA-Farbzahl ≤ 30.

## Claims

1. A process for continuously preparing 2-pyrrolidone by reacting gamma-butyrolactone with ammonia in the liquid phase in the presence of water, which comprises carrying out the reaction at a temperature of from 275 to 300°C and an absolute pressure of from 140 to 180 bar in a molar ratio of the starting materials, gamma-butyrolactone, ammonia and water of 1 : (2 to 4) : (1 to 3).

2. The process according to claim 1, wherein the reaction is carried out at a temperature of from 280 to 295°C.

3. The process according to any of the preceding claims, wherein the reaction is carried out at an absolute pressure of from 150 to 170 bar.

4. The process according to any of the preceding claims, wherein the molar ratio of the starting materials, gamma-butyrolactone, ammonia and water, is 1 : (2.1 to 3.5) : (1.5 to 2.8).

5. The process according to any of the preceding claims, wherein the reaction is carried out in one stage.

6. The process according to any of the preceding claims, wherein the reaction is carried out in a vertical tubular reactor.

7. The process according to the preceding claim, wherein the reaction is carried out in the liquid phase mode.

8. The process according to either of the two preceding claims, wherein the gamma-butyrolactone and an ammonia/water mixture are fed separately to the tubular reactor in the reactor bottom by a two-material injector.

9. The process according to any of the preceding claims, wherein the reaction is carried out in the absence of a catalyst.

10. The process according to any of the preceding claims, wherein, after the reaction, first excess ammonia, then water and finally the 2-pyrrolidone are distilled out of the reaction product.

11. The process according to any of the preceding claims, wherein, after the reaction, the excess ammonia is distilled out of the reaction product in the presence of hydroxides of the metals Na, K, Li, Ba or Ca, in particular in the presence of NaOH.

12. The process according to any of the preceding claims for preparing 2-pyrrolidone having a selectivity of > 93%.

13. The process according to any of the preceding claims for preparing 2-pyrrolidone having a purity of > 99.5%.

14. The process according to any of the preceding claims for preparing 2-pyrrolidone having an APHA color number ≤ 30.

## Revendications

1. Procédé de préparation continue de 2-pyrrolidone par réaction de gamma-butyrolactone avec de l'ammoniac en phase liquide, en présence d'eau, **caractérisé en ce qu'**on effectue la réaction à une température de 275 à 300°C et à une pression absolue de 140 à 180 bars et **en ce que** le rapport molaire entre les matières mises en oeuvre, la gamma-butyrolactone, l'ammoniac et l'eau, est de 1/(2 à 4)/(1à 3).

2. Procédé suivant la revendication 1, **caractérisé en ce qu'**on effectue la réaction à une température de 280 à 295°C.

3. Procédé suivant l'une des revendications précédentes, **caractérisé en ce qu'**on effectue la réaction à une pression absolue de 150 à 170 bars.

4. Procédé suivant l'une des revendications précédentes, **caractérisé en ce que** le rapport molaire entre les matières mises en oeuvre, la gamma-butyrolactone, l'ammoniac et l'eau, est de 1/(2,1 à 3,5)/(1,5 à 2,8).

5. Procédé suivant l'une des revendications précédentes, **caractérisé en ce qu'**on effectue la réaction en une étape.

6. Procédé suivant l'une des revendications précédentes, **caractérisé en ce qu'**on effectue la réaction dans un réacteur tubulaire en position droite.

7. Procédé suivant la revendication précédente, suivant un mode opératoire en fond de cuve.

8. Procédé suivant l'une des deux revendications précédentes, **caractérisé en ce qu'**on amène séparément au réacteur tubulaire la gamme-butyrolactone et un mélange d'ammoniac/eau au fond du réacteur, par un injecteur à deux matières.

9. Procédé suivant l'une des revendications précédentes, **caractérisé en ce qu'**on effectue la réaction en l'absence d'un catalyseur.

10. Procédé suivant l'une des revendications précédentes, **caractérisé en ce que**, après la réaction, on sépare par distillation du produit réactionnel tout d'abord de l'ammoniac excédentaire, ensuite de l'eau et finalement la 2-pyrrolidone.

11. Procédé suivant l'une des revendications précédentes, **caractérisé en ce que**, après la réaction, on sépare par distillation du produit réactionnel l'ammoniac excédentaire en présence d'hydroxydes des métaux Na, K, Li, Ba ou Ca, en particulier en présence de NaOH.

12. Procédé suivant l'une des revendications précédentes pour la préparation de 2-pyrrolidone présentant une sélectivité de > 93 %.

13. Procédé suivant l'une des revendications précédentes pour la préparation de 2-pyrrolidone présentant une pureté de > 99,5 %.

14. Procédé suivant l'une des revendications précédentes pour la préparation de 2-pyrrolidone présentant un indice colorimétrique APHA < 30.
